# EUROPEAN PATENT APPLICATION

(11) **EP 4 737 903 A2**
(43) Date of publication of application: **06.05.2026**
(21) Application number: 26165071.7
(22) Date of filing: 10.12.2021
(51) Int. Cl.: G01N 33/92

(54) **METHOD FOR DETERMINING A LIKELIHOOD OF A SUBJECT TO RESPOND TO LIPID LOWERING THERAPY**

(30) Priority: 11.12.2020 FI 20206284
(62) Divisional of application: 21827604.6
(71) Applicant: MONCYTE Health Oy, 00290 Helsinki (FI)
(72) Inventor: IKONEN, Elina, 00290 Helsinki (FI); PFISTERER, Simon, 00290 Helsinki (FI)
(74) Representative: Papula Oy

(57) **Abstract**

A method for determining a likelihood of a subject to respond to lipid lowering therapy is disclosed. The method may comprise determining a quantitative value of the ability of cells obtained from a biological sample of the subject to take up low-density lipoprotein (LDL), a quantitative value of the expression of LDL receptor (LDLR) in the cells, a quantitative value of the lipid storage capability of the cells, and/or a quantitative value of the lipid mobilization capability of the cells; wherein the quantitative value of the ability of the cells to take up LDL, the quantitative value of the expression of LDLR in the cells, the quantitative value of the lipid storage capability of the cells, and/or the quantitative value of the lipid mobilization capability of the cells is/are indicative of the likelihood of the subject to respond to lipid lowering therapy.

## Description

### TECHNICAL FIELD

The present disclosure relates to a method for determining a likelihood of a subject to respond to lipid lowering therapy and to a method for determining whether a subject is at risk of developing or having atherosclerosis and/or a cardiovascular disease.

### BACKGROUND

Hypercholesterolemia, i.e. an elevated serum cholesterol level in the blood, is currently a leading cause for cardiovascular disease (CVD) in many geographical areas. The regulation of serum cholesterol levels is controlled by dietary intake, endogenous production, and cellular metabolism.

Statins are often used as the first line medication for subjects suffering from hypercholesterolemia. However, often subjects do not respond or achieve a target cholesterol level immediately or in the long term. Some patients do not adhere to statin therapy. Further, a significant portion of patients who have had a heart attack have a second attack within one year of the first attack. Thus there may be a need to lower the cholesterol level of such patients without waiting for a prolonged period to see if they respond to statin treatment. Further, most cholesterol lowering treatments and therapies affect cellular mechanisms, and therefore it may be highly beneficial to be able to quantify such cellular mechanisms.

### SUMMARY

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter.

A method for determining a likelihood of a subject to respond to lipid lowering therapy is disclosed. The method may comprise determining a quantitative value of the ability of cells obtained from a biological sample of the subject to take up low-density lipoprotein (LDL), a quantitative value of the expression of LDL receptor (LDLR) in the cells, a quantitative value of the lipid storage capability of the cells, and/or a quantitative value of the lipid mobilization capability of the cells;
wherein the quantitative value of the ability of the cells to take up LDL, the quantitative value of the expression of LDLR in the cells, the quantitative value of the lipid storage capability of the cells, and/or the quantitative value of the lipid mobilization capability of the cells is/are indicative of the likelihood of the subject to respond to lipid lowering therapy.

A method for determining whether a subject is at risk of developing or having atherosclerosis and/or a cardiovascular disease is also disclosed. The method may comprise determining a quantitative value of the ability of cells obtained from a biological sample of the subject to take up low-density lipoprotein (LDL), a quantitative value of the expression of LDL receptor (LDLR) in the cells, a quantitative value of the lipid storage capability of the cells, and/or a quantitative value of the lipid mobilization capability of the cells;
wherein the quantitative value of the ability of the cells to take up LDL, the quantitative value of the expression of LDLR in the cells, the quantitative value of the lipid storage capability of the cells, and/or the quantitative value of the lipid mobilization capability of the cells is/are indicative of the likelihood of the subject having an increased risk of developing or having atherosclerosis and/or the cardiovascular disease.

### BRIEF DESCRIPTION OF THE DRAWINGS

The accompanying drawings, which are included to provide a further understanding of the invention and constitute a part of this specification, illustrate embodiments and together with the description help to explain the principles of the invention. In the drawings:

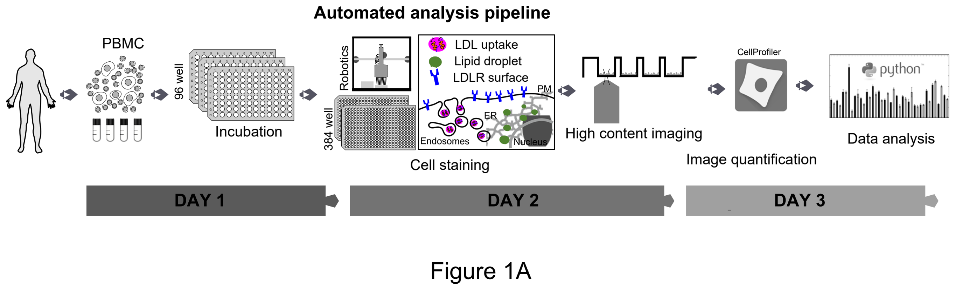

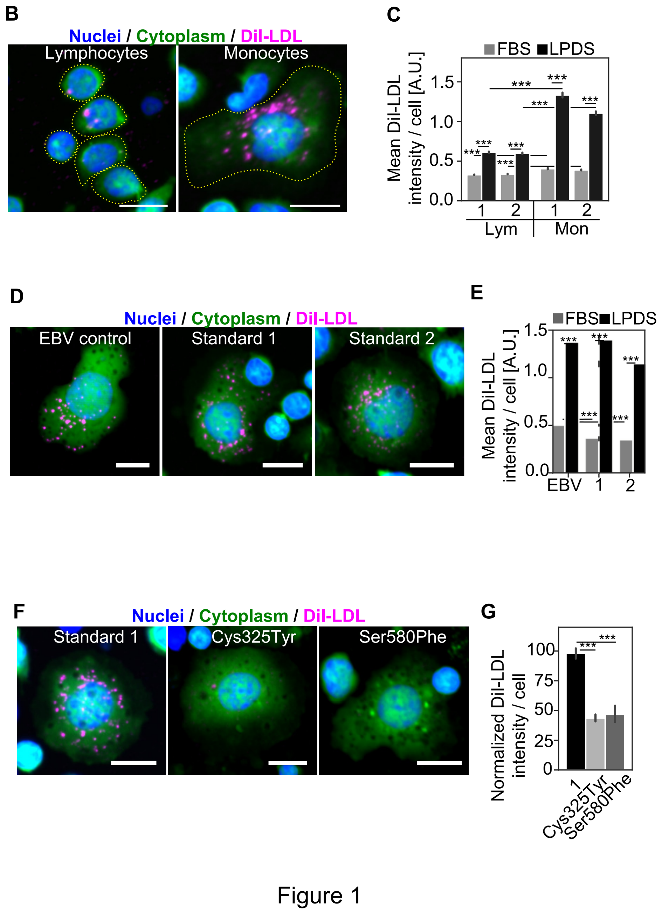

**Figure 1****:** Automated analysis pipeline for multiplex quantification of functional defects in PBMCs. **A)** Schematic presentation of the automated sample processing and image analysis pipeline. For each experiment cryopreserved PBMC samples were thawed, aliquoted into 96 wells and incubated overnight with 10% FBS (complete medium) or 5% LPDS (lipid starvation medium). Cells were labeled with fluorescent LDL (DiI-LDL) or directly transferred to 384 well imaging plates, automatically fixed, stained and subjected to automated high-content imaging. Images were quantified with CellProfiler and single cell data was processed with Python tools. **B)** Representative images showing DiI-LDL uptake in lymphocyte and monocyte populations after treatment with 5% LPDS. **C)** Automated quantification of mean DiI-LDL intensities in lymphocyte and monocyte populations from two standards **(1, 2)** each consisting of a mix of PBMCs from four individuals. n > 74000 lymphocytes and > 16100 monocytes for each treatment and standard from 10 experiments. **D)** DiI-LDL uptake in control EBV lymphoblasts (EBV) and monocytes after LPDS treatment. Representative images are shown. **E)** Quantification of mean DiI-LDL intensities in EBV lymphoblasts and monocytes from two standards as in **(C),** n > 20 000 for EBV lymphoblasts, monocytes as in **(C)** from 10 independent experiments. **F)** Representative images from DiI-LDL uptake in monocytes isolated from familiar hypercholesterolemia (FH) patients with *LDLR* mutations Cys325Tyr or Ser580Phe and standard **(1)** after treatment with 5% LPDS. **G**)Quantification of monocyte DiI-LDL intensities, treated as in **(F)**. n = 3954 cells for standard 1, 1717 cells for Cys325Tyr and 787 cells for Ser580Phe *** < 0.001, scale bar = 10 µm, error bars = 95% confidence interval (CI);

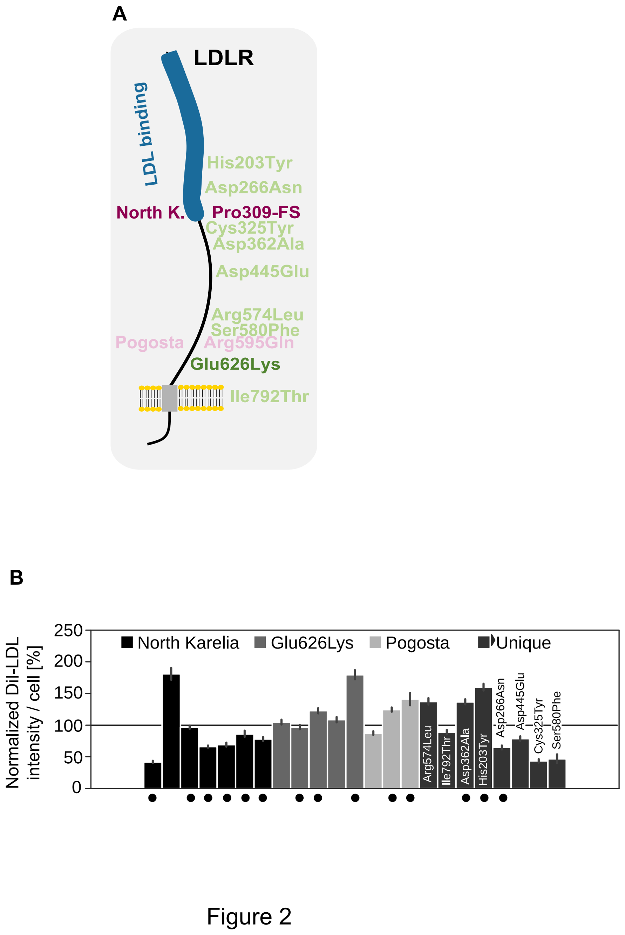

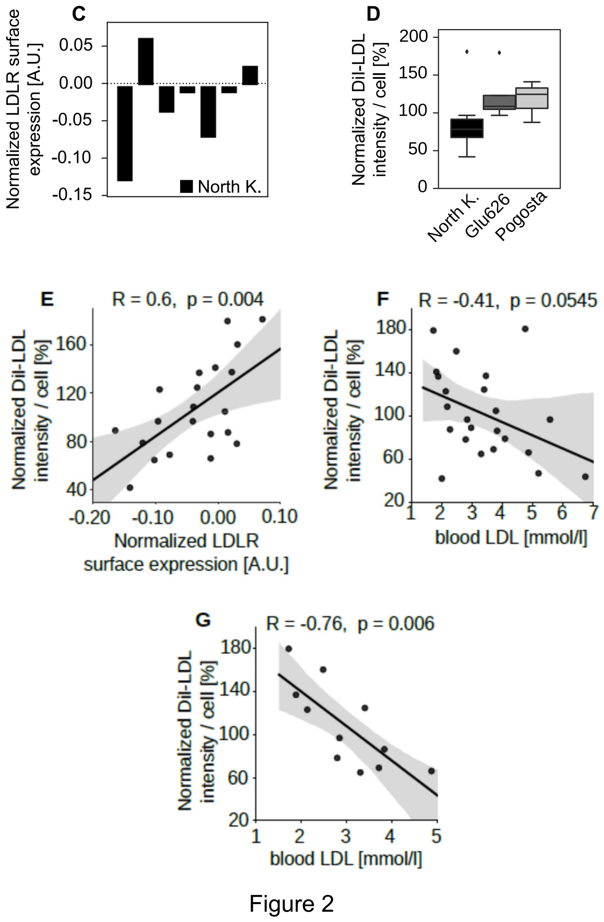

**Figure 2****:** Heterogeneous LDL uptake and LDLR surface expression in He-FH patients' monocytes. **A)** Schematic presentation of *LDLR* mutations included in the examples. **B)** Quantification of monocyte DiI-LDL intensities from FH patients after 5% LPDS treatment, normalized to two standards (100%). On average 5820 monocytes were quantified for each patient in 1-3 experiments. Black dots indicate statin recipients. Error bars = 95% CI. Cys325Tyr and Ser580Phe are from Fig. 1G. **C)** Quantification of monocyte LDLR surface expression relative to two standards, shown in same order as in (**B**). **D)** Box plot of monocyte DiI-LDL intensities for *LDLR* variants North Karelia (n = 7), Glu626Ala (n = 5) and Pogosta (n = 3). **E)** Correlation of monocyte LDLR surface expression and DiI-LDL intensities. **F)** Correlation of monocyte DiI-LDL intensities with blood LDL cholesterol concentrations. **G)** Correlation of monocyte DiI-LDL intensities with LDL-cholesterol plasma levels for patients on statin monotherapy. Grey areas in scatter plots indicate 95% CI;

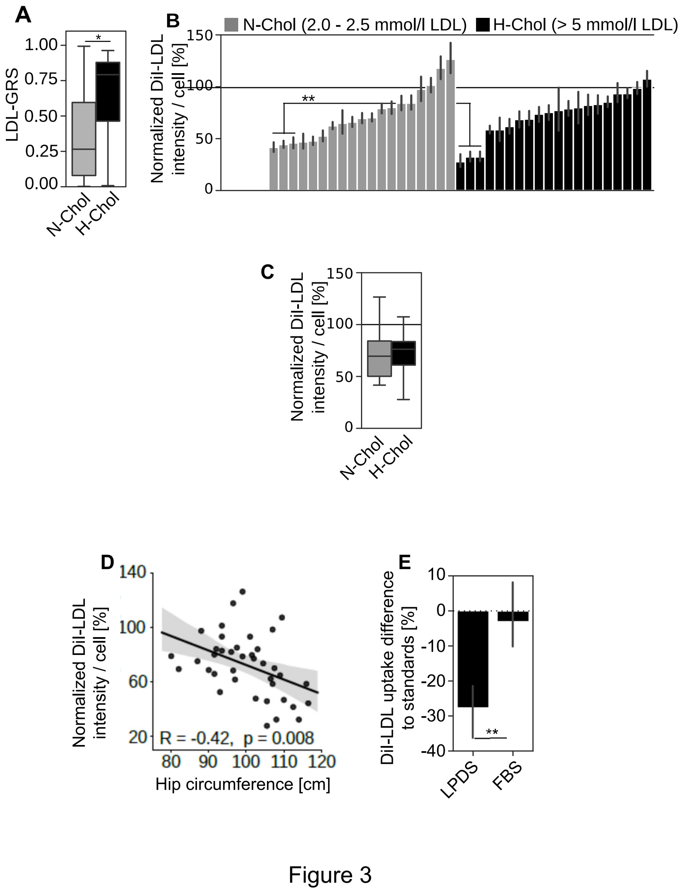

**Figure 3****:** Monocyte LDL uptake profiles in non-FH individuals with normal blood LDL (2-2.5 mmol/l LDL, N-Chol) and with elevated LDL(> 5 mM mmol/l, H-Chol). **A)** Box plot of genetic risk scores for high blood LDL concentration (LDL-GRS) for N-Chol and H-Chol subgroups. NChol n = 18, H-Chol n = 19. * = 0.05. **B)** Quantification of monocyte DiI-LDL intensities in N-Chol and H-Chol subgroups after treatment with 5% LPDS, normalized to standards. On average 1260 monocytes were analysed per patient. **C)** Box plot of monocyte DiI-LDL intensities for N-Chol and H-Chol subgroups quantified in (B), N-Chol n = 19, H-Chol n = 20. **D)** Correlation of monocyte DiI-LDL uptake (after lipoprotein starvation) and hip circumference. **E)** Difference in monocyte LDL uptake for N-Chol and H-Chol individuals relative to standards quantified after 5% LPDS and 10% FBS treatment, n = 39, ** = 0.01. Error bars for bar plots = 95% CI, grey areas in scatter plot indicate 95% CI;

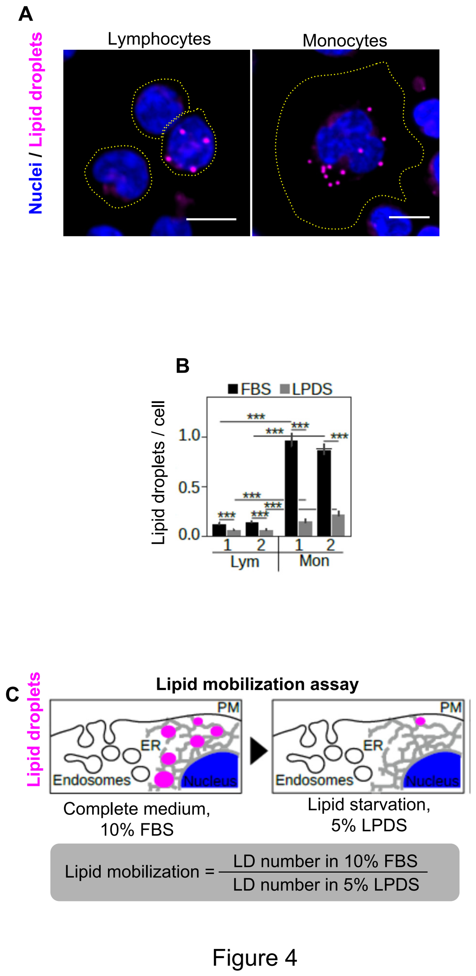

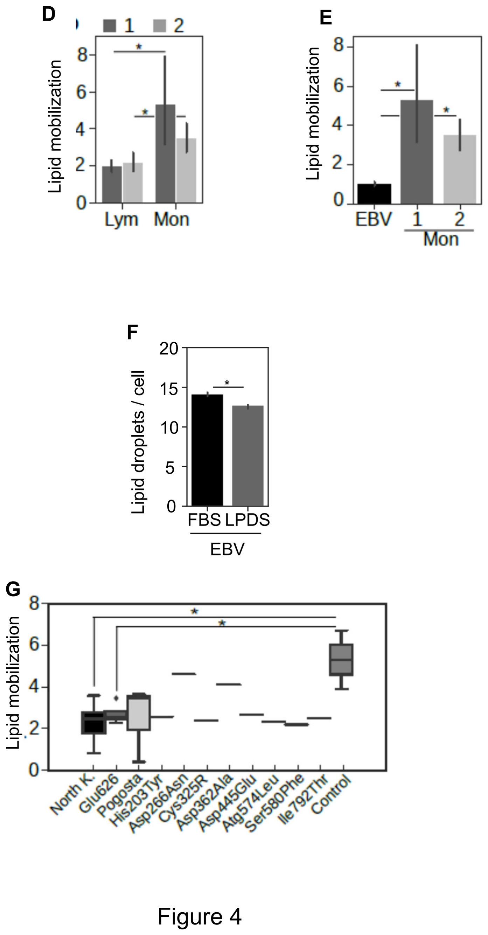

**Figure 4****:** Lipid mobilization assay. **A)** Representative images showing lipid droplets (LDs) in lymphocyte and monocyte populations after treatment with 10% FBS. **B)** Automated quantification of LDs in lymphocyte and monocyte populations after treatment with 10% FBS or 5% LPDS for standard 1 and 2, n = >26000 lymphocytes and >7900 monocytes for each treatment and standard from 8 independent experiments. **C)** Schematic depiction of the lipid mobilization assay. Upon treatment with 10% FBS, cells store surplus lipids in cytosolic LDs. Lipid starvation (5% LPDS) leads to lipid utilization and reduction of LD numbers. Lipid mobilization is calculated by dividing LD numbers after treatment with 10% FBS with LD numbers quantified after treatment with 5% LPDS. **D)** Quantification of lipid mobilization in lymphocyte and monocyte populations for standards 1 and 2, n = 8. **E)** Comparison of lipid mobilization in control EBV lymphoblasts and monocytes from standards 1 and 2 as in (D), n = 8. **F)** Quantification of LD abundance in control EBV lymphoblasts after treatment with 10% FBS or 5% LPDS, n = 7477 monocytes for 10% FBS and 6838 monocytes for 5% LPDS from 8 independent experiments. **G)** Box plot presentation of lipid mobilization scores for FH patients. (North K. n = 7, Glu626Ala n = 5, Pogosta n = 3, other variants n = 1). On average more than 2000 monocytes were analysed per treatment and patient. Error bars for bar plots = 95% CI, scale bar = 10 µm;

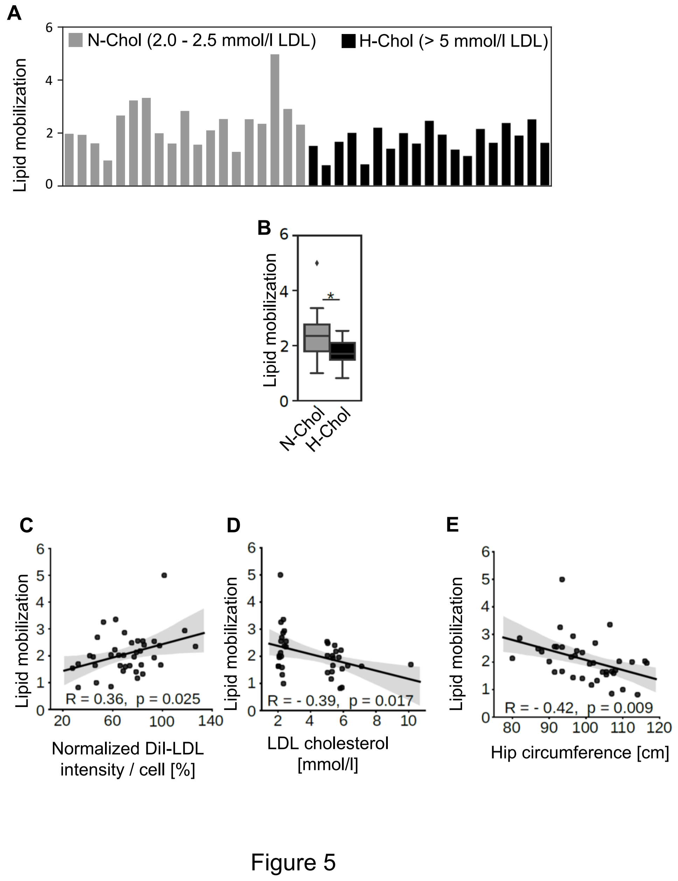

**Figure 5****:** Monocyte lipid mobilization correlates with LDL uptake and is reduced in donors with elevated blood LDL. **A)** Lipid mobilization in monocytes from controls (N-Chol, LDL 2-2.5 mmol/l) and individuals with elevated LDL (H-Chol, LDL > 5 mmol/l and increased polygenic burden), sorted according to monocyte DiI-LDL uptake (Figure 3B). **B)** Box plot of lipid mobilization for N-Chol and H-Chol subgroups, N-Chol n = 19, H-Chol n = 20, * = 0.05. Correlation of monocyte lipid mobilization with DiI-LDL uptake potential (after 5% LPDS treatment) **(C),** blood LDL concentration **(D)** or hip circumference **(E).** Grey areas in scatter plots = 95% CI;

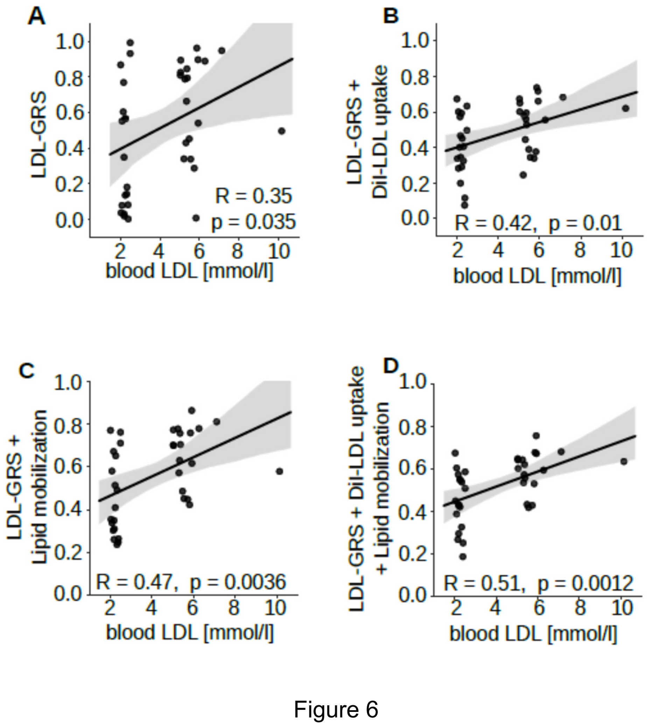

**Figure 6****:** Hybrid scores combining genetic and functional data improve correlations with blood LDL. **A)** Correlation of the genetic risk score for elevated blood LDL concentration (LDL-GRS) with actual blood LDL cholesterol concentrations for biobank donors. **B)** Combination of LDL-GRS with monocyte DiI-LDL uptake potential (after treatment with 5% LPDS) and correlation with blood LDL cholesterol levels. **C)** Combination of LDL-GRS with monocyte lipid mobilization and correlation with LDL cholesterol levels in blood. **D)** Generation of a triple hybrid score consisting of LDL-GRS, monocyte DiI-LDL uptake (after 5% LPDS) and lipid mobilization and correlation of this hybrid score with blood LDL cholesterol concentration. n = 37. Grey areas in scatter plots = 95% CI;

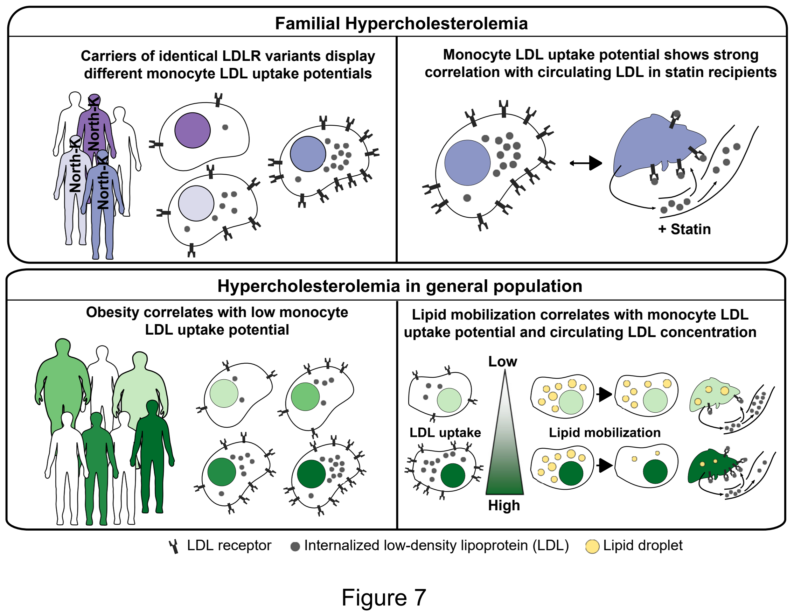

**Figure 7****:** Schematic illustration of functional readouts in monocytes and their correlation with physiological outcomes in monogenic and polygenic hypercholesterolemia; and

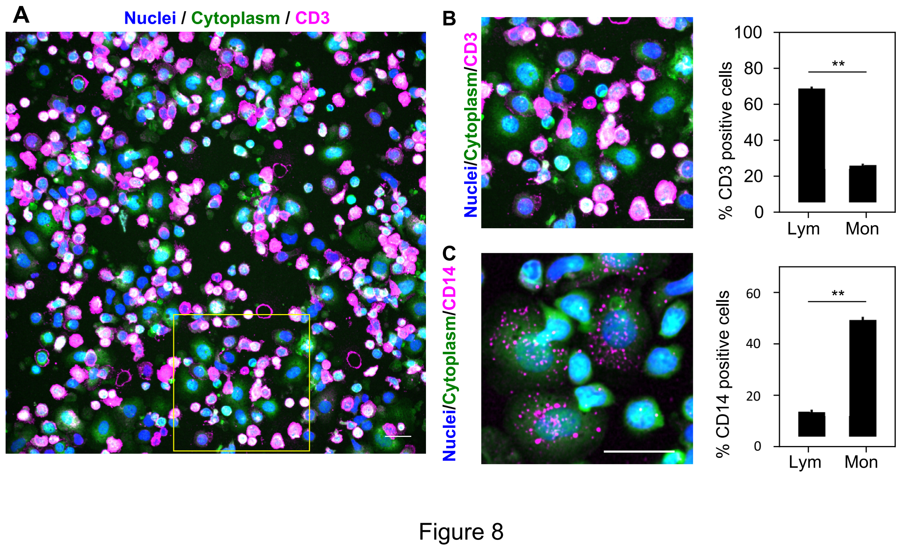

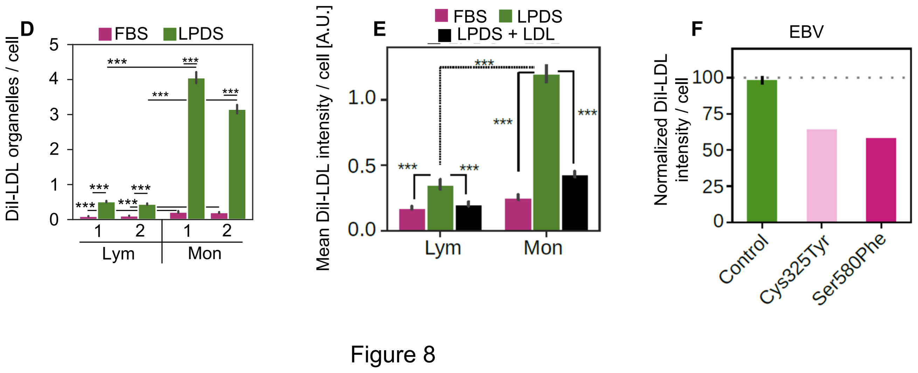

**Figure 8****:** Simultaneous quantification of lymphocyte and monocyte populations and specificity of LDL uptake. **A)** Representative image of PBMC cells stained with DAPI (nuclei), CellMask Green (cytoplasm) and anti-CD3 antibodies (lymphocytes). **B)** Zoom in view of the yellow area in (A) and automated quantification of % CD3 positive cells for cells with a cell area below 115 µm² (designated as lymphocyte population) and above 115 µm² (designates as monocyte population), n = 2 standards, each containing PBMCs from 4 individuals **C)** Representative zoom in image of anti-CD14 stained PBMCs and quantification of % CD14 positive cells in lymphocyte (Lym) and monocyte (Mon) populations as defined in (B), n = 2 standards. **D)** Automated quantification of DiI-LDL positive organelles in lymphocyte and monocyte populations from two standards (1, 2) after treatment with 10% FBS or 5% LPDS. n > 74000 lymphocytes and > 16100 monocytes for each treatment and standard from 10 experiments. **E)** Quantification of DiI-LDL intensities for lymphocytes and monocyte populations after treatment with 10% FBS, 5% LPDS or 5% LPDS supplemented with 100 µg / ml native LDL during DiI-LDL uptake phase (LPDS+LDL). **F)** Quantification of cellular DiI-LDL intensities in EBV lymphoblasts from a control and two familial hypercholesterolemia patients (FH) after 72 h of 5% LPDS treatment. Error bars = 95% CI.

### DETAILED DESCRIPTION

According to a first aspect, a method for determining a likelihood of a subject to respond to lipid lowering therapy is disclosed. The method may comprise
determining a quantitative value of the ability of cells obtained from a biological sample of the subject to take up low-density lipoprotein (LDL), a quantitative value of the expression of LDL receptor (LDLR) in the cells, a quantitative value of the lipid storage capability of the cells, and/or a quantitative value of the lipid mobilization capability of the cells;
wherein the quantitative value of the ability of the cells to take up LDL, the quantitative value of the expression of LDLR in the cells, the quantitative value of the lipid storage capability of the cells, and/or the quantitative value of the lipid mobilization capability of the cells is/are indicative of the likelihood of the subject to respond to lipid lowering therapy.

According to a second aspect, a method for determining whether a subject is at risk of developing or having atherosclerosis and/or a cardiovascular disease is disclosed. The method may comprise
determining a quantitative value of the ability of cells obtained from a biological sample of the subject to take up low-density lipoprotein (LDL), a quantitative value of the expression of LDL receptor (LDLR) in the cells, a quantitative value of the lipid storage capability of the cells, and/or a quantitative value of the lipid mobilization capability of the cells;
wherein the quantitative value of the ability of the cells to take up LDL, the quantitative value of the expression of LDLR in the cells, the quantitative value of the lipid storage capability of the cells, and/or the quantitative value of the lipid mobilization capability of the cells is/are indicative of the likelihood of the subject having an increased risk of developing or having atherosclerosis and/or the cardiovascular disease.

Any embodiments described below in this specification may be understood as relating to either the first aspect, to the second aspect, or to both.

With the method, it may be possible to test and thereby predict a subject's response to lipid lowering therapy, for example to statin, PCSK9, ezetimibe, evinacumab, and/or bempedoic acid treatment.

Therefore, it may be possible to identify relatively quickly subjects who are not likely to respond to a certain lipid lowering treatment, such as statin treatment, alone, such that the subjects may be provided a better working therapy as soon as possible. It may also allow for more subjects to achieve their target lipid levels (for example, cholesterol levels) and/or reduce side effects due to better dosing. Thus the method may allow for reducing the incidence of cardiovascular disease.

In the context of this specification, the terms "therapy" and "treatment" may be understood as commonly in the art. At least in some embodiments, they may be used interchangeably.

In some embodiments, the determining a likelihood of a subject to respond to lipid lowering therapy may comprise or be determining which lipid concentration in the blood of the subject may be achieved by the lipid lowering therapy, and/or a likelihood of the subject to reach a target lipid concentration in the blood of the subject in response to the lipid lowering therapy. In such embodiments, the quantitative value of the ability of the cells to take up LDL, the quantitative value of the expression of LDLR in the cells, the quantitative value of the lipid storage capability of the cells, and/or the quantitative value of the lipid mobilization capability of the cells is/are indicative of the likelihood of the subject to reach a target lipid concentration in the blood of the subject in response to the lipid lowering therapy, and/or indicative of which lipid concentration in the blood of the subject may be achieved by the lipid lowering therapy. The (target) lipid concentration may be e.g. a (target) cholesterol concentration and/or a (target) LDL cholesterol concentration. In such embodiments, it may be possible to determine not only which subjects may respond to the lipid lowering therapy, at least to some extent, but also which subjects may achieve a desired result of the lipid lowering therapy, such as a target lipid concentration.

Any one of, or any combination of, the quantitative value of the ability of the cells to take up LDL, the quantitative value of the expression of LDLR in the cells, the quantitative value of the lipid storage capability of the cells, and/or the quantitative value of the lipid mobilization capability of the cells may be sufficient for determining the likelihood of the subject to respond to lipid lowering therapy, or for determining whether the subject is at risk of developing or having atherosclerosis and/or a cardiovascular disease.

Determining the quantitative value of the ability of cells obtained from the biological sample of the subject to take up low-density lipoprotein (LDL) and/or determining the quantitative value of the expression of LDL receptor (LDLR) in the cells, either alone or in combination, may be considered as determining a quantitative value of LDLR activity in the cells. LDL uptake may, at least in some embodiments, be a better measure of LDLR activity in the cells; however, they may be combined to obtain a more robust readout.

In an embodiment, the method comprises
determining a quantitative value of the ability of cells obtained from a biological sample of the subject to take up low-density lipoprotein (LDL);
wherein the quantitative value of the ability of the cells to take up LDL is indicative of the likelihood of the subject to respond to lipid lowering therapy and/or of having an increased risk of developing or having atherosclerosis and/or the cardiovascular disease.

In an embodiment, the method comprises
determining a quantitative value of the expression of LDL receptor (LDLR) in the cells;
wherein the quantitative value of the expression of LDLR in the cells is indicative of the likelihood of the subject to respond to lipid lowering therapy and/or of having an increased risk of developing or having atherosclerosis and/or the cardiovascular disease.

In an embodiment, the method comprises
determining a quantitative value of the lipid storage capability of the cells;
wherein the quantitative value of the lipid storage capability of the cells is indicative of the likelihood of the subject to respond to lipid lowering therapy and/or of having an increased risk of developing or having atherosclerosis and/or the cardiovascular disease.

In an embodiment, the method comprises
determining a quantitative value of the lipid mobilization capability in the cells;
wherein the quantitative value of the lipid mobilization capability is indicative of the likelihood of the subject to respond to lipid lowering therapy and/or of having an increased risk of developing or having atherosclerosis and/or the cardiovascular disease.

In an embodiment, the method comprises
determining a quantitative value of the ability of cells obtained from a biological sample of the subject to take up low-density lipoprotein (LDL) and a quantitative value of the expression of LDL receptor (LDLR) in the cells;
wherein the quantitative value of the ability of the cells to take up LDL and the quantitative value of the expression of LDLR in the cells are indicative of the likelihood of the subject to respond to lipid lowering therapy and/or of having an increased risk of developing or having atherosclerosis and/or the cardiovascular disease.

In an embodiment, the method comprises
determining a quantitative value of the ability of cells obtained from a biological sample of the subject to take up low-density lipoprotein (LDL) and a quantitative value of the lipid storage capability of the cells;
wherein the quantitative value of the ability of the cells to take up LDL and the quantitative value of the lipid storage capability of the cells are indicative of the likelihood of the subject to respond to lipid lowering therapy and/or of having an increased risk of developing or having atherosclerosis and/or the cardiovascular disease.

In an embodiment, the method comprises
determining a quantitative value of the ability of cells obtained from a biological sample of the subject to take up low-density lipoprotein (LDL) and a quantitative value of the lipid mobilization capability of the cells;
wherein the quantitative value of the ability of the cells to take up LDL and the quantitative value of the lipid mobilization capability of the cells are indicative of the likelihood of the subject to respond to lipid lowering therapy and/or of having an increased risk of developing or having atherosclerosis and/or the cardiovascular disease.

In an embodiment, the method comprises
determining a quantitative value of the expression of LDL receptor (LDLR) in cells obtained from a biological sample of the subject and a quantitative value of the lipid storage capability of the cells;
wherein the quantitative value of the expression of LDLR in the cells and the quantitative value of the lipid storage capability of the cells are indicative of the likelihood of the subject to respond to lipid lowering therapy and/or of having an increased risk of developing or having atherosclerosis and/or the cardiovascular disease.

In an embodiment, the method comprises
determining a quantitative value of the expression of LDL receptor (LDLR) in cells obtained from a biological sample of the subject and a quantitative value of the lipid mobilization capability of the cells;
wherein the quantitative value of the expression of LDLR in the cells and the quantitative value of the lipid mobilization capability of the cells is/are indicative of the likelihood of the subject to respond to lipid lowering therapy and/or of having an increased risk of developing or having atherosclerosis and/or the cardiovascular disease.

In an embodiment, the method comprises
determining a quantitative value of the lipid storage capability of cells obtained from a biological sample of the subject and a quantitative value of the lipid mobilization capability of the cells;
wherein the quantitative value of the lipid storage capability of the cells and the quantitative value of the lipid mobilization capability of the cells are indicative of the likelihood of the subject to respond to lipid lowering therapy and/or of having an increased risk of developing or having atherosclerosis and/or the cardiovascular disease.

In an embodiment, the method comprises
determining a quantitative value of the ability of cells obtained from a biological sample of the subject to take up low-density lipoprotein (LDL), a quantitative value of the expression of LDL receptor (LDLR) in the cells, and a quantitative value of the lipid storage capability of the cells;
wherein the quantitative value of the ability of the cells to take up LDL, the quantitative value of the expression of LDLR in the cells, and the quantitative value of the lipid storage capability of the cells are indicative of the likelihood of the subject to respond to lipid lowering therapy and/or of having an increased risk of developing or having atherosclerosis and/or the cardiovascular disease.

In an embodiment, the method comprises
determining a quantitative value of the ability of cells obtained from a biological sample of the subject to take up low-density lipoprotein (LDL), a quantitative value of the lipid storage capability of the cells, and a quantitative value of the lipid mobilization capability of the cells;
wherein the quantitative value of the ability of the cells to take up LDL, the quantitative value of the lipid storage capability of the cells, and the quantitative value of the lipid mobilization capability of the cells are indicative of the likelihood of the subject to respond to lipid lowering therapy and/or of having an increased risk of developing or having atherosclerosis and/or the cardiovascular disease.

In an embodiment, the method comprises
determining a quantitative value of the expression of LDL receptor (LDLR) in cells obtained from a biological sample of the subject, a quantitative value of the lipid storage capability of the cells, and a quantitative value of the lipid mobilization capability of the cells;
wherein the quantitative value of the expression of LDLR in the cells, the quantitative value of the lipid storage capability of the cells, and the quantitative value of the lipid mobilization capability of the cells are indicative of the likelihood of the subject to respond to lipid lowering therapy and/or of having an increased risk of developing or having atherosclerosis and/or the cardiovascular disease.

In an embodiment, the method comprises
determining a quantitative value of the ability of cells obtained from a biological sample of the subject to take up low-density lipoprotein (LDL), a quantitative value of the expression of LDL receptor (LDLR) in the cells, and a quantitative value of the lipid mobilization capability of the cells;
wherein the quantitative value of the ability of the cells to take up LDL, the quantitative value of the expression of LDLR in the cells, and the quantitative value of the lipid mobilization capability of the cells are indicative of the likelihood of the subject to respond to lipid lowering therapy and/or of having an increased risk of developing or having atherosclerosis and/or the cardiovascular disease.

In an embodiment, the method comprises
determining a quantitative value of the ability of cells obtained from a biological sample of the subject to take up low-density lipoprotein (LDL), a quantitative value of the expression of LDL receptor (LDLR) in the cells, a quantitative value of the lipid storage capability of the cells, and a quantitative value of the lipid mobilization capability of the cells;
wherein the quantitative value of the ability of the cells to take up LDL, the quantitative value of the expression of LDLR in the cells, the quantitative value of the lipid storage capability of the cells, and the quantitative value of the lipid mobilization capability of the cells are indicative of the likelihood of the subject to respond to lipid lowering therapy and/or of having an increased risk of developing or having atherosclerosis and/or the cardiovascular disease.

The method may comprise comparing the quantitative value of the ability of the cells to take up LDL to a quantitative value of control cells or to a control value, wherein a decrease in the quantitative value of the ability of the cells to take up LDL is indicative of a decreased likelihood of the subject to respond to lipid lowering therapy and/or of having an increased risk of developing or having atherosclerosis and/or the cardiovascular disease, and/or wherein an increase in the quantitative value of the ability of the cells to take up LDL is indicative of an increased likelihood of the subject to respond to lipid lowering therapy and/or a reduced likelihood of having atherosclerosis and/or the cardiovascular disease.

The method may comprise comparing the quantitative value of the expression of LDL receptor (LDLR) in the cells to a quantitative value of control cells or to a control value, wherein a decrease in the quantitative value of the expression of LDLR in the cells is indicative of a decreased likelihood of the subject to respond to lipid lowering therapy and/or of having an increased risk of developing or having the cardiovascular disease, and/or wherein an increase in the quantitative value of the expression of LDLR in the cells is indicative of an increased likelihood of the subject to respond to lipid lowering therapy and/or a reduced likelihood of having atherosclerosis and/or the cardiovascular disease.

Identification of subjects having an increased likelihood of the subject to respond to lipid lowering therapy, i.e. good responders to lipid lowering therapy, may be useful in that if the subject does not subsequently respond to the therapy, it may indicate that the subject has not adhered to the therapy. If a subject is a good responder, it may also allow lower levels of medication (e.g. a lower dose of statin) to achieve a desired effect, and thereby reduction of potential side effects and/or improved adherence to the medication.

The method may comprise comparing the quantitative value of the lipid storage capability of the cells to a quantitative value of control cells or to a control value;
wherein an increase in the quantitative value of the lipid storage capability of the cells is indicative of a decreased likelihood of the subject to respond to lipid lowering therapy and/or of having an increased risk of developing or having atherosclerosis and/or the cardiovascular disease, and/or wherein a decrease in the quantitative value of the lipid storage capability of the cells is indicative of an increased likelihood of the subject to respond to lipid lowering therapy and/or a reduced likelihood of having atherosclerosis and/or the cardiovascular disease.

The method may comprise comparing the quantitative value of the lipid mobilization capability of the cells to a quantitative value of control cells or to a control value;
wherein a decrease in the quantitative value of the lipid mobilization capability of the cells is indicative of a decreased likelihood of the subject to respond to lipid lowering therapy and/or of having an increased risk of developing or having atherosclerosis and/or the cardiovascular disease, and/or wherein an increase in the quantitative value of the lipid mobilization capability of the cells is indicative of an increased likelihood of the subject to respond to lipid lowering therapy and/or a reduced likelihood of having atherosclerosis and/or the cardiovascular disease.

The quantitative value of the ability of the cells to take up LDL may be determined e.g. by contacting the cells with labelled LDL particles; incubating the resulting mixture thereby obtainable for a period sufficient to allow cellular internation-alization of the labelled LDL particles; and determining the amount and/or the number of labelled LDL particles internalized by the cells. Alternatively or additionally, the intensity of the labelled LDL particles internalized by the cells and/or the quantification of organelles containing the labelled LDL particles may be used to determine the quantitative value of the ability of the cells to take up LDL. The labelled LDL particles may bind to an LDL receptor (or LDL receptors) on the surface of the cells, such that a complex is formed by the labelled LDL particles and the LDL receptor. The complex may then be internalized into the cells. However, other methods capable of measuring the ability of the cells to take up LDL may be contemplated, for example methods utilizing ApoB (apolipoprotein B) or artificial LDL molecules.

The quantitative value of the expression of LDLR in the cells may comprise or be surface expression and/or cellular expression of LDLR. Expression of LDLR may be determined e.g. by using an anti-LDLR antibody or other ligand capable of binding LDLR. However, other methods capable of measuring the expression of LDLR in the cells may be contemplated, for example methods quantifying *LDLR* mRNA abundance (e.g. by quantitative PCR or by RNA sequencing).

The quantitative value of the lipid storage capability may be determined e.g. by determining the quantitative value (e.g. the amount or relative amount, area, intensity and/or the number) of lipid droplets (LDs) in the cells (i.e. cellular lipid droplets).

The quantitative value of the lipid mobilization capability of the cells may be determined e.g. by determining the quantitative value of lipid droplets (LDs) in the cells in complete medium relative to the quantitative value of lipid droplets in the cells in lipoprotein depleted medium. This may be done e.g. by calculating a ratio of the quantitative value of lipid droplets in the cells in complete medium relative to the quantitative value of lipid droplets in the cells in lipoprotein depleted medium. The term "complete medium" may be understood as referring to a cell culture medium containing all components required for the maintenance and/or growth of the cells. An example of a suitable complete medium is a medium containing 10% FBS (fetal bovine serum). An example of a lipoprotein depleted medium is a comparable medium (i.e. a medium comparable to a corresponding complete medium) that does not contain FBS but contains 5% LPDS (lipoprotein deficient serum).

The quantitative value of lipid droplets (LDs) in the cells and quantitative value of the lipid storage capability may be determined by measuring the abundance of lipid droplets in intracellular lipid storage organelles. The cells may be immobilized e.g. to a plastic or glass substrates. Then the cells may be fixed and stained with a nuclear stain and a hydrophobic fluoro-phore which accumulates inside lipid droplets. Then the cells may be subjected to automatic imaging; software tools may be used to identify the cells and individual lipid droplets in each cell. This may allow for determining the exact number of cellular lipid droplets, their size and/or their fluorescent intensity. Details of the method have been described e.g. by Pfisterer et al., Nature Communications 2017; 8:14858. Based on this information it is possible to quantify the lipid mobilization.

However, other methods for determining the quantitative value of the lipid mobilization capacity of the cells may be contemplated, for example, biochemical determination of lipids under different conditions, or flow cytometry may be used to quantify LD intensities under the different conditions. Alternatively, the lipid mobilization may be measured by first exposing the cells to a high lipid load to stimulate a large number of lipid droplets and then switch to complete medium which contains less lipids, thereby monitoring lipid mobilization. With automated microscopy and image analysis it may be possible to achieve a more accurate quantification of lipid droplets. Flow cytometry could also be used to measure the cellular lipid droplet intensity, but not necessarily the number and the sizes of the lipid droplets. Nonetheless, the flow cytometry could be used to quantify intensity changes under different conditions and thus permit the calculation of lipid mobilization. Also, lipid droplets could be quantified by antibody stainings for a lipid droplet associated protein. Alternatively, cellular lipid content could be measured with biochemical methods, such as cellular lipid extraction with solvents and quantification via thin layer chromatography or mass spectrometry.

In the context of this specification, the term "lipid mobilization capacity" may be understood as referring to the capacity of the cells to utilize stored lipids. In an embodiment, the term "lipid mobilization capacity" may be understood as referring to a ratio and/or fold difference of lipid droplet numbers, cellular lipid droplet area, and/or cellular lipid droplet intensity in complete medium relative to lipid droplet numbers, cellular lipid droplet area, and/or cellular lipid droplet intensity in lipoprotein depleted medium (e.g. in the same or comparable cells). However, the quantitative value of the lipid mobilization capacity may, alternatively or additionally, be determined by determining the difference between lipid droplet numbers, cellular lipid droplet area, and/or cellular lipid droplet intensity in complete medium and in lipoprotein depleted medium.

The biological sample of the subject may be a blood sample or a sample derived from a blood sample, e.g. a buffy coat sample. Other biological samples may be contemplated, for example cell samples derived from tissues such as fibroblasts, keratinocytes and/or mesenchymal stem cells.

The cells (i.e. the cells obtained from the biological sample of the subject) may comprise or be peripheral blood mononuclear cells (PBMCs). PBMCs may be obtained from the biological sample, such as a blood sample or buffy coat sample, e.g. by density gradient centrifugation. PBMCs may also be selected by positive or negative antibody selection techniques.

The cells may, alternatively or additionally, comprise or be e.g. lymphocytes immortalized with Epstein-Barr virus (EBV lymphoblasts); neutrophils; and/or granulocytes.

The cells may be cryopreserved.

The subject may be human, but the subject may, additionally or alternatively, be an animal, such as a mammalian, for example, a primate, a non-human primate, a dog, a cat, a horse, a sheep, a goat, a bovine, a rabbit, a pig and/or a rodent, such as a mouse or a rat, or any other species.

The subject may have hyperlipidemia. In an embodiment, the subject has hypercholesterolemia and/or hypertriglyceridemia.

In the method according to the second aspect, the cardiovascular disease may comprise or be e.g. at least one of coronary artery disease, such as angina or myocardial infarction, stroke, heart attack, cerebrovascular disease or peripheral artery disease.

The subject may, in some embodiments, be receiving the lipid lowering therapy. In other words, the subject may be or has been on the lipid lowering therapy at the time the biological sample is obtained or has been obtained from the subject. In other embodiments, the biological sample may be obtainable or obtained from a subject that has not yet received lipid lowering therapy.

The lipid lowering therapy may comprise or be statin therapy. Statins are often used as the first line medication for subjects suffering from hypercholesterolemia. However, often subjects do not respond or achieve a target cholesterol level immediately or in the long term when receiving statin therapy. Some subjects do not adhere to statin therapy. Further, it has been observed that a significant portion of subjects who have had a heart attack have a second attack within one year of the first attack. Thus there may be a need to lower the cholesterol level(s) of such subjects without waiting for a prolonged period to see if they respond to statin therapy. Additionally or alternatively, the lipid lowering therapy may comprise or be PCSK9 inhibitor therapy, ezetimibe therapy, evinacumab therapy, and/or bempedoic acid therapy. Other possible therapies, treatments or lipid lowering agents may be contemplated or developed in the future.

The method may be performed using automated image and data analysis. The method may be performed using high content imaging followed by automated image and data analysis. The high content imaging may refer to or comprise automated microscopy, such as automated fluorescence microscopy. The automated microscopy may include e.g. automated wide-field fluorescence microscopy and/or confocal microscopy. High content imaging may allow better automation and/or flexibility. It may also reduce the time required for the method.

The method may be performed using a robotic platform.

However, other methods may, alternatively or additionally, be contemplated. For example, the method may be performed using flow cytometry and/or non-automated microscopy.

The cells may be immobilized on a surface. For example, they may be immobilized on the surface for high content imaging. The cells may be immobilized on the surface as a monolayer. Suitable surfaces may include e.g. plastic and/or glass surfaces, such as high-content imaging plates. The immobilization may allow for storage and repeated analyses (e.g. image acquisition) of the (same) cells after storage. It may allow for grouping and/or discriminating between two or more cell populations. The immobilization may also prevent cell aggregation and allow for automation of staining and/or imaging of the cells. Immobilized cells may be suitable for imaging at a subcellular resolution that may enable determining the quantitative amount of lipid droplets and/or internalized LDL particles. The immobilization may also allow for the determination of the subcellular localization of the lipid droplets and/or internalized LDL particles.

The immobilized cells may adhere to the surface. To facilitate adhesion, the surface may be selected, treated and/or prepared such that it allows for improved adhesion. Suitable surfaces may be coated. Suitable coatings may include e.g. coatings that may facilitate cell adhesion to the surface, such as a Poly-D lysine (PDL), Poly-L-lysine, collagen, and/or laminin coatings.

The cells may be grouped to two or more (i.e. at least two) cell populations, and/or one or more cell populations may be selected from the cells, and the quantitative value of the ability to take up LDL, the quantitative value of the expression of LDLR, the quantitative value of the lipid storage capability, and/or the quantitative value of the lipid mobilization capability of at least one of the cell populations may be determined separately from the other cell populations. For this, the cells may be immobilized on a surface. Immobilizing the cells may however not be necessary. For example, the cells may be grouped to two or more cell populations e.g. by an antibody staining using an antibody specific for a cell population or other type of staining capable of differentiating between the two or more cell populations, and/or by their morphological properties. For example, the at least one cell population may be a cell population exhibiting the highest signal to noise ratio (as compared to the other cell population(s) within the cells). As another example, PBMC cells may be grouped to monocytes and lymphocytes (and optionally to other cell types), and the quantitative value(s) may be determined separately for the monocytes, lymphocytes, and any other optional cell populations. Monocytes and lymphocytes may be discriminated from each other based on size, as monocytes may spread out after adhesion to a surface, such as a coated imaging plate, resulting in an enlarged cytoplasmic region. However, at least in some embodiments, the quantitative value(s) determined separately for the monocytes, lymphocytes, and/or any other optional cell types or populations, may be combined and used e.g. in correlations.

However, grouping the cells to two or more cell populations may not be necessary for determining the quantitative value of the ability to take up LDL, the quantitative value of the expression of LDLR, the quantitative value of the lipid storage capability, and/or the quantitative value of the lipid mobilization capability of the cells. For example, the quantitative value(s) may be determined from an entire PBMC population (or a part thereof).

The quantitative value of the ability of the cells of the subject to take up LDL may be determined using labelled LDL particles. However, other means which may allow for the determination of the ability of the cells of the subject to take up LDL may also be contemplated. The LDL particles may be e.g. fluorescently or radioactively labelled. The LDL particles may be preserved e.g. by freezing.

The quantitative value of the ability of the cells of the subject to take up LDL may be determined using LDL particles preserved by snap freezing. The snap freezing may be done e.g. by lowering the temperature of the LDL particles to a temperature of -70 °C or lower very quickly, for example for a time period of at least 1 s, or at least 5 s, or at least 10 s, or at least 30 s, or at least 1 min. After the snap freezing, the LDL particles thus preserved may be stored e.g. at a temperature of about -70 to about -80 °C; however, they may also be stored at other temperatures, such as temperatures below about -10 °C or below about -20 °C, or in the range of about -10 °C to about -20 °C. The snap freezing and optionally storing the LDL particles at a suitable temperature may prevent the aggregation or modification of the LDL particles, which could alter the measurement of the quantitative value of the ability of the cells to take up LDL. Thus the LDL particles may be stored longer and/or provide more reliable results.

The quantitative value of the ability of the cells to take up LDL, the quantitative value of the expression of LDLR, the quantitative value of the lipid storage capability, and/or the quantitative value of the lipid mobilization capability may be normalized using a standard comprising a mixture of cells obtainable from biological samples of a plurality of subjects. The plurality of subjects may comprise or be e.g. at least 2, or at least 3, or at least 4, or at least 5, or at least 10 subjects, or at least 100 subjects. Thus it may be possible to obtain a standard that is more reproducible and/or more uniform than e.g. standards obtainable from a biological sample of a single person.

Large-scale control standards may be used to normalize the data. New control standards may be compared to previous control standards to allow comparison of samples over a long timeframe. The different overlapping control standards may be measured e.g. in 3 or more experiments to have enough values so as to compare them reliably. For example, this may be done by providing a mixed preparation of PBMCs from e.g. 4 or more individuals. These standards may be aliquoted and frozen so that each aliquot is sufficient for one experiment. For each test or experiment a new aliquot may be used. This may ensure that the standard is of the same or similar quality for each experiment. Once a new standard is made, the standard may be compared to a previous control standard, by quantifying e.g. LDL uptake and lipid droplet abundance after treatment with control medium and lipoprotein depleted medium. The background of the cells may also be quantified. For example, only standards which are similar in regard to background, LDL uptake and lipid droplet abundance to the previous standards may be considered as suitable standards for upcoming experiments. For long term comparability of experimental results, it may be beneficial to compare results obtained from one standard to results obtained with a different standard. Therefore, the different standards may be measured in multiple experiments to reduce effects from experimental variation.

The method may further comprise administering a treatment to the subject having a decreased likelihood to respond to lipid lowering therapy to thereby treat the subject in order to prevent or treat hypercholesterolemia in the subject. The treatment may comprise or be administration of a statin, ezetimibe, evinacumab, bempedoic acid, and/or a PCSK9 inhibitor. In an embodiment, the subject has a decreased likelihood to respond to statin therapy.

The treatment may comprise or be administration of a statin, ezetimibe, evinacumab, bempedoic acid, and/or a PCSK9 inhibitor. The treatment may comprise or be e.g. administration of a co-treatment, for example a co-treatment including a statin and ezetimibe, or a co-treatment including a statin and a PCSK9 inhibitor. The treatment may be different from the lipid lowering therapy, to which the subject has a decreased likelihood to respond. For example, if the subject is determined to have a decreased likelihood to respond to statin treatment (and wherein the subject may or may not already be receiving a lipid lowering therapy, such as statin therapy, at the time the biological sample is obtained or has been obtained from the subject), the treatment may comprise or be e.g. administration of a co-treatment including a statin and ezetimibe, or a co-treatment including a statin and a PCSK9 inhibitor. Other possible co-treatments may be contemplated or developed in the future.

In embodiments in which the subject is receiving a lipid lowering therapy (i.e. the subject may be or has been on the lipid lowering therapy at the time the biological sample is obtained or has been obtained from the subject), the treatment may be a co-treatment, i.e. it may comprise or be an additional lipid lowering treatment. The additional lipid lowering treatment may then be a different and/or an additional treatment from the lipid lowering therapy the subject is already receiving.

Results obtainable by the method may be combined with other measures to improve the determination of the likelihood of the subject to respond to lipid lowering therapy, and/or whether the subject is at risk of developing or having atherosclerosis and/or the cardiovascular disease.

The method may further comprise calculating a risk score based on the quantitative value of the ability of the cells to take up LDL, the quantitative value of the expression of LDLR, the quantitative value of the lipid storage capability, and/or the quantitative value of the lipid mobilization capability.

Various other characteristics, measures or values may be included in the calculation of the risk score. Such other characteristics, measures or values may include e.g. one or more of the following: age, gender, dietary habits, physical measurements such as body mass index (BMI), hip circumference, waist circumference, blood metabolites, circulating lipoprotein concentrations, genetic risk factors, family history and/or previous medical history. Such other characteristics, measures or values may be determined prior to, simultaneously, or after the biological sample is obtained from the subject. As a skilled person will understand, some of the characteristics, measures or values may be information collected e.g. using a questionnaire or clinical data collected earlier. Some of the characteristics, measures or values may be determined (or may have been determined) by biochemical or clinical diagnostic measurements and/or medical diagnosis.

The method may further comprise determining whether the subject is at risk of developing or having atherosclerosis and/or the cardiovascular disease on the basis of at least one genetic marker associated with atherosclerosis and/or the cardiovascular disease or the risk thereof, and/or the method further comprises calculating a risk score based on the presence of at least one genetic marker associated with atherosclerosis and/or the cardiovascular disease or the risk thereof and on the quantitative value of the ability of the cells to take up LDL, the quantitative value of the expression of LDLR, the quantitative value of the lipid storage capability, and/or the quantitative value of the lipid mobilization capability. Genetic analysis, i.e. using the at least one genetic marker, may improve the determination of whether the subject is at risk of developing or having atherosclerosis and/or the cardiovascular disease.

In an embodiment, the method comprises calculating a risk score based on the presence of at least one genetic marker associated with atherosclerosis and/or the cardiovascular disease or the risk thereof and on the quantitative value of the ability of the cells to take up LDL, and the quantitative value of the lipid mobilization capability. The risk score may, at least in some embodiments, be a polygenic risk score. In the context of this specification, the abbreviation "GRS" may be understood as referring to a genetic risk score.

The at least one genetic marker associated with the cardiovascular disease or the risk thereof, or the risk score, may comprise or be e.g. a marker for familiar hypercholesterolemia; a mutation in an LDL receptor gene (*LDLR*); a mutation in apolipoprotein-B gene (*APOB*); a mutation in LDLRAP1 (Low Density Lipoprotein Receptor Adaptor Protein 1, ARH); a mutation in proprotein convertase subtilisin kexin type 9 gene (*PCSK9*); and/or a polygenic risk score, such as LDL-GRS; GRS for hypertriglyceridemia; and/or GRS for cardiovascular disease.

A composition comprising a mixture of labelled LDL particles is also disclosed. The LDL particles in the mixture may be obtainable from a plurality of individual subjects, and the composition may be preserved by snap freezing the composition and/or the LDL particles prior or after the labelling. In other words, the composition may be obtainable by preserving it and/or the LDL particles by snap freezing prior or after the labelling. This may be referred to as cryopreservation of the LDL particles. In the context of the composition, the LDL particles may be any LDL particles according to one or more embodiments described in this specification.

The LDL particles obtainable from e.g. plasma samples of the plurality of individual subjects may be first quality checked before they are pooled e.g. for the labelling. For example, LDL particles from samples with a very high lipid content, e.g. based on a visual inspection and/or quantification of lipoprotein concentration(s), may be excluded from the mixture. This is because such LDL particles may not necessarily perform well.

The method may comprise normalizing the quantitative value of the ability of the cells to take up LDL using the composition.

The following embodiments are disclosed:
1. A method for determining a likelihood of a subject to respond to lipid lowering therapy, wherein the method comprises
   determining a quantitative value of the ability of cells obtained from a biological sample of the subject to take up low-density lipoprotein (LDL), a quantitative value of the expression of LDL receptor (LDLR) in the cells, a quantitative value of the lipid storage capability of the cells, and/or a quantitative value of the lipid mobilization capability of the cells;
   wherein the quantitative value of the ability of the cells to take up LDL, the quantitative value of the expression of LDLR in the cells, the quantitative value of the lipid storage capability of the cells, and/or the quantitative value of the lipid mobilization capability of the cells is/are indicative of the likelihood of the subject to respond to lipid lowering therapy.
2. A method for determining whether a subject is at risk of developing or having atherosclerosis and/or a cardiovascular disease, wherein the method comprises
   determining a quantitative value of the ability of cells obtained from a biological sample of the subject to take up low-density lipoprotein (LDL), a quantitative value of the expression of LDL receptor (LDLR) in the cells, a quantitative value of the lipid storage capability of the cells, and/or a quantitative value of the lipid mobilization capability of the cells;
   wherein the quantitative value of the ability of the cells to take up LDL, the quantitative value of the expression of LDLR in the cells, the quantitative value of the lipid storage capability of the cells, and/or the quantitative value of the lipid mobilization capability of the cells is/are indicative of the likelihood of the subject having an increased risk of developing or having atherosclerosis and/or the cardiovascular disease.
3. The method according to embodiment 1, wherein the method comprises comparing the quantitative value of the ability of the cells to take up LDL to a quantitative value of control cells or to a control value, wherein a decrease in the quantitative value of the ability of the cells to take up LDL is indicative of a decreased likelihood of the subject to respond to lipid lowering therapy, and/or wherein an increase in the quantitative value of the ability of the cells to take up LDL is indicative of an increased likelihood of the subject to respond to lipid lowering therapy.
4. The method according to any one of embodiments 1 - 3, wherein the cells comprise or are peripheral blood mononuclear cells (PBMCs).
5. The method according to any one of embodiments 1 - 4, wherein the cells comprise or are monocytes.
6. The method according to any one of embodiments 1 - 5, wherein the subject has hyperlipidemia.
7. The method according to any one of embodiments 2 - 6, wherein the cardiovascular disease comprises or is at least one of coronary artery disease, such as angina or myocardial infarction, stroke, heart attack, cerebrovascular disease, or peripheral artery disease.
8. The method according to any one of embodiments 1 - 7, wherein the lipid lowering therapy comprises or is statin therapy, PCSK9 inhibitor therapy, ezetimibe therapy, evinacumab therapy, and/or bempedoic acid therapy.
9. The method according to any one of embodiments 1 - 8, wherein the method is performed using high content imaging followed by automated image and data analysis.
10. The method according to any one of embodiments 1 - 9, wherein the cells are immobilized on a surface, optionally as a monolayer.
11. The method according to any one of embodiments 1 - 10, wherein the cells are grouped to two or more cell populations, and the quantitative value of the ability to take up LDL, the quantitative value of the expression of LDLR, the quantitative value of the lipid storage capability, and/or the quantitative value of the lipid mobilization capability of at least one of the cell populations is/are determined separately from the other cell populations.
12. The method according to any one of embodiments 1 - 11, wherein the quantitative value of the ability of the cells of the subject to take up LDL is determined using labelled LDL particles, wherein the LDL particles are optionally fluorescently or radioactively labelled.
13. The method according to any one of embodiments 1 - 12, wherein the quantitative value of the ability of the cells of the subject to take up LDL is determined using LDL particles preserved by snap freezing.
14. The method according to any one of embodiments 1 - 13, wherein the quantitative value of the ability of the cells to take up LDL, the quantitative value of the expression of LDLR, the quantitative value of the lipid storage capability, and/or the quantitative value of the lipid mobilization capability is/are normalized using a standard comprising a mixture of cells obtainable from biological samples of a plurality of subjects.
15. The method according to any one of embodiments 2 - 14, wherein the method further comprises determining whether the subject is at risk of developing or having the cardiovascular disease on the basis of at least one genetic marker associated with the cardiovascular disease or the risk thereof, and/or the method further comprises calculating a risk score based on the presence of at least one genetic marker associated with the cardiovascular disease or the risk thereof and on the quantitative value of the ability of the cells to take up LDL, the quantitative value of the expression of LDLR, the quantitative value of the lipid storage capability, and/or the quantitative value of the lipid mobilization capability.
16. The method according to any one of embodiments 1 - 15, wherein
   the method is performed using high content imaging followed by automated image and data analysis;
   the cells are immobilized on a surface, optionally as a monolayer;
   the cells are grouped to two or more cell populations, and the quantitative value of the ability to take up LDL, the quantitative value of the expression of LDLR, the quantitative value of the lipid storage capability, and/or the quantitative value of the lipid mobilization capability of at least one of the cell populations is/are determined separately from the other cell populations; and
   the quantitative value of the ability of the cells to take up LDL, the quantitative value of the expression of LDLR, the quantitative value of the lipid storage capability, and/or the quantitative value of the lipid mobilization capability is/are normalized using a standard comprising a mixture of cells obtainable from biological samples of a plurality of subjects.
17. The method according to embodiment 16, wherein the quantitative value of the ability of the cells of the subject to take up LDL is determined using LDL particles preserved by snap freezing.
18. The method according to embodiment 16 or 17, wherein the cells comprise or are peripheral blood mononuclear cells (PBMCs), the PBMC cells are grouped to monocytes, lymphocytes and optionally to other cell types, and the quantitative value(s) are determined separately for the monocytes, lymphocytes, and any other optional cell populations.
19. A composition comprising a mixture of labelled LDL particles, wherein the LDL particles in the mixture are obtainable from a plurality of individual subjects, and the composition is preserved by snap freezing the composition and/or the LDL particles prior or after the labelling.

### EXAMPLES

Reference will now be made in detail to various embodiments, an example of which is illustrated in the accompanying drawings.

The description below discloses some embodiments in such a detail that a person skilled in the art is able to utilize the embodiments based on the disclosure. Not all steps or features of the embodiments are discussed in detail, as many of the steps or features will be obvious for the person skilled in the art based on this specification.

### EXAMPLE 1

Various abbreviations and acronyms are used below in the examples as follows.

Apo-B: Apolipoprotein-B; BSA: Bovine serum albumin; EBV: Epstein-Barr virus; CI: Confidence interval; DAPI: Diamidino-2-phenylindole; DiI: 1,1'-dioctadecyl-3,3,3',3'-tetramethylindocarbocyanine perchlorate; DMSO: Dimethyl sulfoxide; FBS: Fetal bovine serum, FH: Familial hypercholesterolemia; HEPES: 4-(2-hydroxyethyl)-1-piperazine ethanesulfonic acid; LD: Lipid droplet; LDL: Low-density lipoprotein; LDL-GRS: LDL genetic risk score; LDLR: Low-density lipoprotein receptor; CVD: Cardiovascular disease; LPDS: Lipoprotein deficient serum; PBMC: Peripheral blood mononuclear cells; PBS: Phosphate buffered saline; PCSK9: Proprotein convertase subtilisin/kexin type 9; PDL: Poly-D-lysine; VLDL: Very low-density lipoprotein.

***Materials:*** Lipoprotein deficient serum (LPDS) was obtained from fetal bovine serum by ultracentrifugation (Goldstein et al., Methods Enzymol. 1983;98:241-260). For DiI-LDL, fresh LDL from human plasma samples was first prepared (Finnish Red Cross permit 39/2016) by density centrifugation (Stephan and Yurachek, J Lipid Res. 1993;34:325-330) and then labelled with 1,1'-dioctadecyl-3,3,3',3'-tetramethyl-indocarbocyanine perchlorate (DiI) (Reynolds et al., Am J Pathol. 1985;121:200-211). 4',6-diamidino-2-phenylindole (DAPI), Poly-D lysine (PDL) and Histopacque Premium were obtained from Sigma. DiI, anti-mouse Alexa 568, HCS CellMask Deep Red and HCS CellMask Green where obtained from Thermo Fisher. Mouse anti-LDLR (clone 472413) was from R&D systems.

***Peripheral blood mononuclear cells (PBMC) and blood samples:*** He-FH patients were identified in the Metabolic Syndrome in Men study (Laakso et al., J Lipid Res. 2017;58:481-493) and blood samples obtained during patient follow-up in accordance with the declaration of Helsinki regarding experiments involving humans. Two He-FH patients (Cys325Tyr and Ser580Phe) for which obtained PBMC and EBV lymphoblast cell samples were obtained were described previously (Romano et al., J Lipid Res. 2011;52:2095-2100). PBMC samples from the Finnish population survey, FINRISK 2012, and the donor linked data (including genotypes) were obtained from THL Biobank (www.thl.fi/biobank) and used under the Biobank agreements no (2016_15, 2016_117 and 2018_15). The donors with elevated LDL levels (LDL > 5 mM) and normocholesterolemic (LDL 2.0-2.5 mM) study groups from the biobank were age, gender and BMI matched. The donors in either of the groups did not have cholesterol lowering medication by the time of sampling, and based on a food frequency questionnaire, did not receive an elevated proportion of energy intake as saturated or trans-fat. Buffy coat samples from healthy blood donors were obtained from the Finnish Red Cross (permit 392016).

***Cell* culture:** Control EBV lymphoblasts (GM14664) were obtained from Coriell Cell Repository and cultured in RPMI-1640 supplemented with 15% FBS, penicillin/streptomycin (100 U/ ml each) and 2 mM L-Glutamine. For continuous culturing of EBV lymphoblasts, 3x10⁶ cells were transferred to 5 ml of fresh medium once a week. Single use cryopreserved EBV lymphoblast aliquots were used for experiments. Cells were cryopreserved in 70% PBMC medium (RPMI-1640, penicillin/streptomycin, 2 mM L-glutamine, 1 mM sodium pyruvate, and 1 mM HEPES), 20% FBS and 10% DMSO.

***PBMC isolation:*** Blood or buffy coat samples were mixed 1:1 with phosphate buffered saline (PBS) including 2.5 mM EDTA (PBS-E). The blood mixture was gently layered over Histopacque Premium (1.0073, for mononuclear cells) and centrifuged 40 min at 400 g. The PBMC cell layer was removed, transferred to a new 15 ml reaction tube and mixed with PBS-E. Cells were centrifuged at 400 g for 10 min and incubated in 2 ml of red blood cell lysis buffer for 1 min (155 mM NH₄Cl, 12 mM NaHCO₃, 0.1 mM EDTA). 10 ml of PBS-E was added and cells were pelleted and washed with PBS-E. Then cells were resuspended in 5 ml PBMC medium (RPMI-1640, penicillin/streptomycin, 2 mM L-glutamine, 1 mM sodium pyruvate, and 1 mM HEPES), counted, pelleted and resuspended in freezing medium (70% PBMC-medium, 20% FBS, 10 % DMSO) and cryopreserved in liquid nitrogen.

***Cell treatments, DiI-LDL uptake, transfer to imaging plates and fixation:*** Cryopreserved EBV lymphoblasts or PBMCs were thawed in PBMC medium, and centrifuged at 400 g for 10 min. The cells were resuspended in PBMC medium and transferred to wells of a 96 well plate containing FBS (10% final concentration) or LPDS (5% final concentration) and incubated for 24 h. For DiI-LDL uptake experiments 30 µg/ml DiI-LDL was added for 1 h at 37°C. Subsequently, cells were transferred to conical 96 well plates and centrifuged at 400 g for 10 min. Using a robotic platform (Opentrons, New York, USA) medium was removed and cells were resuspended in PBMC medium. Cells were centrifuged, automatically resuspended in PBMC medium and transferred to PDL coated 384 well high-content imaging plates (Corning). After 30 min of incubation at 37°C cells were automatically fixed with 4% paraformaldehyde in 250 mM HEPES, 1 mM CaCl₂, 100 µM MgCl₂, pH 7.4 and washed with PBS. For lipid droplet and LDLR surface stainings, cells were directly transferred to PDL coated 384 well high-content plates, adhered, automatically fixed and washed with PBS.

***Lipid droplet analyses:*** Cells were processed as described before (Pfisterer et al., Nat Commun. 2017;8:14858) with the following changes: Fixed cell samples were automatically stained with 1 µg/ml LD540 (Princeton BioMolecular Research) and 5 µg/ml DAPI. 3D stacks of optical slices were acquired automatically either with a Nikon Eclipse Ti-E inverted microscope equipped with a 40 × Planfluor objective with NA 0.75 and 1.5 zoom. Or with a PerkinElmer Opera Phenix High Content Imaging system with a 63x water immersion objective, NA 1.15. Image stacks were automatically deconvolved either with Huygens software (Scientific Volume Imaging, b.v.) or a custom made Python tool based on the open source tools PSF generator and deconvolution lab. Maximum intensity projections were made from the deconvolved image stacks with custom Python tools. These tools can be accessed via: https://github.com/lopaavol/Oputils. Automated quantification of lipid droplets was performed as described previously (Pfisterer et al., Nat Commun. 2017;8:14858; Salo et al., Dev Cell. 2019;50:478-493.e9; Vanharanta et al., Traffic. 2020;21:386-397). Lipid droplet counts were normalized to the average of two control standards which were included in each imaging plate.

***LDLR surface staining:*** All staining procedures were performed automatically. Fixed cells were quenched with 50 mM NH₄Cl for 15 min and washed twice with PBS. Cells were incubated with block solution (PBS, 1% BSA) for 10 min followed by staining with mouse anti-LDLR in block solution for 60 min. Cells were washed three times with PBS followed by incubation with secondary antibody solution (anti-mouse-Alexa 568, DAPI 5 µg/ml and HCS CellMask Green stain 0.25 µg/ml) for 45 min at room temperature. Cells were washed with PBS and 3D stacks of optical slices were acquired for DAPI (nuclei), CellMask Green (cytoplasm), Alexa 568 (LDLR surface) and Alexa 640 (background) channels using an Opera Phenix high-content imaging system with a 40x water immersion objective NA 1.1. LDLR surface and background images were automatically deconvolved with custom build Python deconvolution tools and maximum intensity projections were made. The resulting images were automatically analysed with CellProfiler (Kamentsky et al., Bioinformatics. 2011;27:1179-1180). LDLR surface intensities were background subtracted for each individual cell and normalized by subtracting mean LDLR surface intensities from the two standards, which were included in each imaging plate.

***Quantification of DiI-LDL uptake:*** DiI-LDL labelled and fixed cells (see section cell treatments) were automatically processed with a robotic platform (Opentrons). Cells were stained with 5 µg/ml DAPI and 0.5 µg/ml HCS CellMask Deep Red and image stacks for three channels, DAPI (nuclei), DiI-LDL and CellMask Deep Red (cytoplasm) were acquired. Automated microscopy and single cell quantifications with CellProfiler were performed as described in the section LDLR surface staining. The robotic work flow for washing and transferring DiI-LDL labeled PBMCs from multiple patients resulted in defined DiI-LDL intensity levels for individual columns of an imaging plate, which was determined with standards and combined with plate normalizations to make mean cellular DiI-LDL intensities and DiI-LDL organelle counts comparable from experiment to experiment.

***LDL genetic risk score:*** The LDL GRS was calculated using the LDpred method based on both the previously published GRS by *Talmud* et al. and a European genome-wide association study (GWAS) meta-analysis with 56945 samples (Talmud et al., The Lancet. 2013;381:1293-1301; Vilhjálmsson et al., Am J Hum Genet. 2015;97:576-592). LDL uptake and lipid mobilization parameters were normalized to a range from 0 to 1 to generate uptake and mobilization scores. Hybrid scores represent the average of LDL-GRS and uptake and/or mobilization scores.

***Data analysis:*** Python (Python Software Foundation, www.python.org) was used with the Pandas package (pandas.pydata.org) to analyse single cell data obtained from CellProfiler. With a cytoplasm area below 115 µm² cells were classified as lymphocytes whereas cells with an area above 115 µm² were classified as monocytes. Statistical significance was assessed with SciPy (scipy.org) using the "ttest_inde" function which calculates a two-tailed pvalue for independent samples. Data visualization was performed with Matplotlib (matplotlib.org) and Seaborn (seaborn.pydata.org).

### EXAMPLE 2

### Automated pipeline for multiplex quantification of hypercholesterolemia-related functional defects in primary human cells

An automated analysis pipeline was set up to quantify LDL uptake, LDLR surface expression and lipid storage under different conditions from less than two million peripheral blood mononuclear cells (PBMCs) (2-4 ml blood) in a scalable and robust manner **(****Figure 1A****).** Key requirements were high sensitivity, flexibility, low cost and a high level of automation. These were achieved by shifting experiments to 96 and 384 well plates, using automated liquid handling systems for cell stainings and employing high-content imaging followed by automated image and data analysis **(****Figure 1A****).** Cryopreserved PBMCs were utilized for the analysis. The cells were recovered in 96 well plates and incubated in complete medium (10% FBS) or lipoprotein starvation medium (5% LPDS) for 24 h, followed by automated sample preparation and imaging. To compare quantifications from different experiments, two standards were included for normalization. Each standard was a mix of large-scale PBMC isolations from four healthy blood donors, and the cells were cryopreserved at a defined cell density for one-time use aliquots.

The automated analysis pipeline enables simultaneous quantification of different white blood cell populations **(****Figure 8A-C).** After cell adhesion to coated imaging plates, lymphocytes remain small, as visualized with CD3 surface staining **(****Figure 8A, B).** Monocytes spread out, resulting in an enlarged cytoplasmic region **(****Figure 8A, C).** This allows discrimination of the two cell populations based on size **(****Figure 1B****,** **Figure 8A, C).** First LDL uptake was compared in lymphocyte and monocyte populations. **(****Figure 1B, C).** In complete medium (10% FBS), monocyte and lymphocyte LDL uptake was comparable. Lipoprotein starvation (5% LPDS) increased LDL uptake, as expected, but the increase in DiI-LDL intensity was much more pronounced in monocytes **(****Figure 1C****).** This effect was also observed when quantifying the number of DiI-LDL positive organelles **(****Figure 8D****).** Importantly, DiI-LDL uptake was reversible in lymphocytes and monocytes by adding surplus unlabeled LDL **(****Figure 8E****),** arguing for a saturable, receptor-mediated mechanism.

Due to low signal intensities in primary blood cells, lymphocytes immortalized with Epstein-Barr virus (EBV lymphoblasts) are often a preferred choice for LDL uptake studies. LDL uptake was therefore compared in EBV lymphoblasts and primary monocytes **(****Figure 1D****).** Signal strengths after lipoprotein starvation were roughly similar between the cell types **(****Figure 1E****).** However, monocytes showed lower DiI-LDL intensities in complete medium, translating to more effective stimulation of LDL uptake upon lipoprotein starvation compared to EBV lymphoblasts **(****Figure 1E****).** To further scrutinize the cells, He-FH patient samples for which both EBV lymphoblasts and PBMC samples were available were analysed **(**Figure 1F**, G).** In He-FH patients EBV lymphoblasts, DiI-LDL intensities were decreased compared to control, but the reduction was less obvious than in primary monocytes **(****Figure 8F****).** Overall, the established pipeline provides reliable quantification of DiI-LDL uptake in white blood cells, with monocytes being more suitable than lymphocytes or EBV lymphoblasts for the analyses.

### EXAMPLE 3

### Heterogeneous LDL uptake and LDLR surface expression in He-FH patients with identical LDLR mutations.

Next, the analysis pipeline was used to characterize 21 FH patients from the Metabolic Syndrome in Men (METSIM) cohort study. The subjects comprise nine different *LDLR* variants of which Pro309Lysfs* (FH North Karelia), Glu626Ala, and Arg595Gln (FH Pogosta) were observed in several patients, and His203Tyr represents a novel *LDLR* variant **(****Figure 2A****).** Fourteen of the patients were on statin therapy **(****Figure 2B****,** black dots). First DiI-LDL uptake was quantified in monocytes after lipoprotein starvation. Unexpectedly, LDL uptake was substantially different for individuals bearing identical *LDLR* variants **(****Figure 2B****).** This was most pronounced for the North Karelia *LDLR* variant that generates a truncated LDL receptor **(****Figure 2A****).** Importantly, LDL uptake rates were in line with LDLR surface expression for these individuals **(****Figure 2C****).** This suggests that regulatory mechanisms may enhance the expression of the unaffected *LDLR* allele and/or stabilize the encoded protein. Interestingly, median DiI-LDL intensities for the FH-North Karelia *LDLR* variant were lower compared to the Glu626Ala and FH-Pogosta variants **(****Figure 2D****).** This is in line with higher blood LDL concentrations for FH North Karelia patients than FH Pogosta patients. Overall, there was a significant correlation of LDLR surface expression with cellular LDL uptake after lipoprotein starvation **(****Figure 2E****),** confirming that higher LDLR surface presentation is a key determinant of LDL uptake. Higher monocyte LDL uptake tended to correlate with lower blood LDL levels **(****Figure 2F****).** This correlation became highly significant when only statin recipients were analysed **(****Figure 2G****).**

### EXAMPLE 4

### Assessing LDL internalization in non-FH individuals with normal or elevated blood LDL.

A large majority of hypercholesterolemia patients does not carry FH-alleles. Therefore cellular LDL uptake was investigated in PBMCs from 20 biobank donors with elevated LDL levels (blood LDL >5 mM) **(H-Chol)** and 19 with normal LDL levels (blood LDL 2-2.5 mM) **(N-Chol).** DNA sequencing confirmed that common Finnish *LDLR* variants were not present among the subjects. However, a genetic risk score for high blood LDL concentration (LDL-GRS) (Ripatti et al., Circ Genomic Precis Med. 2020;13:e002725) was increased in the H-Chol group **(****Figure 3A****).**

Monocyte DiI-LDL intensities were quantified after lipoprotein starvation for N-Chol and H-Chol individuals **(****Figure 3B****)** and observed major variability in LDL uptake, but overall no significant differences between the N-Chol and H-Chol groups **(****Figure 3C****).** Both groups included persons with severely reduced LDL internalization **(****Figure 3B****),** with the lowest LDL uptake values in HChol individuals. Overall, monocyte LDL uptake after lipoprotein starvation failed to show a negative correlation with blood LDL (data not shown). Low LDL uptake, however, correlated with increased blood LDL levels in the H-Chol subgroup, even though this correlation relied on a single individual with very high blood LDL concentration (data not shown).

To investigate additional factors potentially influencing the heterogenous LDL uptake responses in N-Chol and H-Chol individuals, correlations of two indicators available for obesity, body mass index (BMI) and hip circumference were performed. Strikingly, increased hip circumference correlated with reduced LDL uptake **(****Figure 3D****)** and a similar tendency was observed for BMI (data not shown).

When comparing to the blood donor standards, cells from biobank donors showed on average 30% lower DiI-LDL uptake after lipoprotein starvation **(****Figure 3E****).** Interestingly, this difference was not observed when DiI-LDL uptake was measured in complete medium (10% FBS) where LDLRs are downregulated **(****Figure 3E****).** This suggests that in cells from biobank donors, defects in maximal LDL uptake potential are masked when LDLR is expressed at baseline levels.

### EXAMPLE 5

### Assessment of cellular lipid storage and lipid mobilization in monocytes.

Cells store excess lipids in intracellular lipid droplets (LDs). It was reasoned that when cells have sufficient lipids available, they are less prone to take up lipoproteins. Therefore the quantification of cellular LDs was included in the analysis pipeline, and the system was verified with the standard samples. In complete medium (10% FBS), monocytes showed more LDs than lymphocytes **(**Figure 4A**, B).**

Incubation in lipoprotein depleted medium (5% LPDS) for 24 h resulted in a significant reduction in
the number of LDs in both cell populations, with more pronounced reductions in monocytes **(****Figure 4B****).** Similarly, the LD area decreased, indicating that a reduction in LD numbers was not compensated by forming bigger LDs (data not shown). Of note, the number of LDs in complete medium did not correlate with circulating LDL levels, suggesting that lipid storage in white blood cells *in vitro* is not determined by the cells exposure to circulating LDL before isolation (data not shown).

Next, a lipid mobilization assay was established to measure the cellular capacity to utilize stored lipids. Lipid mobilization was defined as the fold difference of LD numbers in complete medium vs. lipoprotein depleted medium **(****Figure 4C****).** First, lipid mobilization was higher in monocytes than in lymphocytes **(****Figure 4D****).** This might explain why monocytes show a greater increase in LDL internalization upon lipoprotein starvation **(****Figure 1C****).** Second, EBV lymphoblasts and primary monocytes were compared. Whereas monocytes from the standards showed lipid mobilization scores of about 3-5, EBV lymphoblasts scored a value of 1 **(****Figure 4E****).** This indicates that lipid mobilization during 24 h lipoprotein starvation is severely reduced in EBV lymphoblasts. Indeed, EBV lymphoblasts contained 10-fold more LDs in complete medium and LD numbers and total LD area decreased only marginally upon lipoprotein starvation **(****Figure 4F****).** Thus, monocytes are superior to lymphocytes and EBV lymphoblasts not only for analyzing LDL uptake but also for quantifying lipid mobilization.

### EXAMPLE 6

### Lipid mobilization is reduced in hypercholesterolemia patients and correlates with LDL uptake.

In He-FH patients' monocytes, lipid mobilization was significantly reduced in cells expressing the *LDLR* variants North Karelia and Glu626Ala as compared to the standards **(****Figure 4G****).** The cells expressing unique *LDLR* variants also showed reduced lipid mobilization except for Asp266Asn and Asp362Ala variants **(****Figure 4G****).** These data suggest that defective LDLR function often associates with reduced lipid mobilization.

It was then investigated whether the cells from N-Chol and H-Chol biobank donors display differences in lipid mobilization. Analogously to LDL uptake, a large fluctuation in lipid mobilization in both subgroups was observed **(****Figure 5A****).** Interestingly, as a group the H-Chol individuals showed significantly lower lipid mobilization rates as compared to N-Chol subjects **(**Figure 5A**, B).**

This prompted a scrutinization whether lipid mobilization correlates with LDL uptake potential in the biobank donors. It was observed that individuals with higher lipid mobilization also showed higher LDL internalization **(****Figure 5C****).** Moreover, reduced lipid mobilization correlated with higher plasma LDL concentration **(****Figure 5D****).** Remarkably, obesity indicators correlated negatively with lipid mobilization **(****Figure 5E****).** Together, these data speak for an interrelationship of LDL uptake and lipid mobilization in the development of hypercholesterolemia.

### EXAMPLE 7

### Hybrid scores of genetic and functional cell data improve correlations with circulating LDL

The H-Chol biobank donors of the FINRISK population cohort displayed an increased LDL-GRS **(****Figure 3A****)** and LDL-GRS correlated with blood LDL concentration **(****Figure 6A****).** Of note, LDL-GRS did not correlate with LDL uptake after lipoprotein starvation (data not shown), suggesting that LDL-GRS and cellular LDL uptake monitor at least in part distinct processes. Interestingly, combination of LDL-GRS with monocyte DiI-LDL uptake to a novel hybrid score improved the correlation with blood LDL concentration **(****Figure 6B****).** Analogously to LDL uptake, lipid mobilization did not correlate with LDL-GRS (data not shown). However, combination of lipid mobilization with LDL-GRS drastically improved the correlation with blood LDL **(****Figure 6C****).**

Finally, a triple hybrid score of LDL-GRS, lipid mobilization and LDL uptake showed the best correlation with blood LDL levels **(****Figure 6D****).**

In these examples, the multiplexed high-content analysis pipeline established integrates analysis of cellular LDL uptake and quantification of cellular lipid storage in an automated workflow. This provides several advantages over flow-cytometry based assays currently used to quantify LDL uptake in patient's white blood cells: 1) Immobilization of cells to coated surfaces prevents cell aggregation, allowing image acquisition after sample storage and straightforward detection of lymphocyte and monocyte populations. 2) Cell staining procedures can be automated easily. 3) Fewer cells are required, and 4) subcellular resolution enables quantification of lipid droplets and internalized LDL particles.

Fibroblasts, lymphocytes and EBV lymphoblasts are often considered as the cell types of choice for LDL uptake assays. Here it is shown that monocytes can provide larger detection windows than EBV lymphoblasts and yield DiI-LDL uptake signal intensities about twice as high as primary lymphocytes. Furthermore, it was found that monocytes are better suited for the quantification of intracellular lipid storage and lipid mobilization upon lipoprotein depletion.

In the current examples, over 310 conditions (combinations of assays and treatments) were analyzed for 62 patient samples. The processing speed depends on the number of assays and treatments performed for each PBMC sample. In its present stage, the automated pipeline enables 120 conditions (e.g. 30 PBMC samples with two assays and two treatments) to be analyzed in 3 days, when operated by a single person. The throughput can be increased considerably by adding more liquid handling systems to the pipeline and increasing computing resources for image processing.

The lipid mobilization assay was established to investigate how altered regulation of lipid storage might impact cellular LDL uptake and circulating LDL levels. Interestingly, lipid mobilization provided better correlations with blood LDL concentrations than cellular LDL uptake.

Quantification of lipid mobilization may therefore provide additional means to identify individuals at risk for hypercholesterolemia. Furthermore, the interrelationship of lipid mobilization and LDL uptake highlights the added value of combining multiple readouts to elucidate the underlying causes of hypercholesterolemia. This approach might also provide deeper insight into the cellular mechanisms underlying familial combined hyperlipidemia a frequent disorder, which is influenced by metabolic alterations. Obesity is known to predispose to elevated blood LDL levels and increased CVD risk. The results provide evidence that at the mechanistic level, obesity correlates with reduced cellular LDL uptake and lipid mobilization. Interestingly, clinical trials indicate that statins may be less effective in obese individuals. Considering that statins act by blocking cholesterol synthesis and increasing LDL internalization, it is plausible that the reduced LDL uptake and lipid mobilization potential contribute to the lower potency of statins in obese persons.

So far, the added value of quantifying cellular parameters to define risk groups or guide treatment decisions in the clinical management of hypercholesterolemia has not been clear. However, the genuinely high heterogeneity of cellular readouts that were observed in individuals with normal blood LDL concentrations, hypercholesterolemic persons and even He-FH patients carrying identical LDL variants, may provide new possibilities for personalized risk assessment and treatment recommendation.

The present study highlights two situations where this appears feasible. Firstly, it was observed that combining LDL-GRS with the quantification of LDL uptake and lipid mobilization to novel hybrid scores improved the correlation with circulating LDL. Especially for young individuals where clinical manifestations of hypercholesterolemia are not overt, such hybrid scores may improve personalized risk assessment for hypercholesterolemia and CVD. Secondly, it was found a significant correlation of low monocyte LDL uptake with high blood LDL for He-FH patients on statin treatment. This may suggest that monocyte LDL uptake potential may facilitate the anticipation of individual's statin responsiveness. For poor statin responders, co-treatments with ezetimibe or PCSK9 inhibitors might be warranted to achieve blood LDL target levels. Remarkably, response to statin therapy is highly heterogeneous and up to 80% of people with coronary heart disease do not achieve their blood LDL target levels, emphasizing that better intervention strategies are urgently needed.

In summary (see **Figure 7****),** the results revealed that individuals with normal and elevated blood LDL as well as He-FH patients bearing identical *LDLR* variants displayed high variability in cellular LDL uptake and lipid mobilization. Lipid mobilization was significantly reduced in hypercholesterolemia patients and correlated with lower LDL uptake potential and higher blood LDL. Furthermore, reduced LDL uptake and lipid mobilization correlated with obesity indicators.

Importantly, quantification of genetic plus cell-based hybrid scores for individual risk assessment and analysis of LDL uptake in He-FH patients on statin therapy open up new possibilities for personalized medicine in hypercholesterolemia.

It is obvious to a person skilled in the art that with the advancement of technology, the basic idea may be implemented in various ways. The embodiments are thus not limited to the examples described above; instead they may vary within the scope of the claims.

The embodiments described hereinbefore may be used in any combination with each other. Several of the embodiments may be combined together to form a further embodiment. A method, a product, a system, or a use, disclosed herein, may comprise at least one of the embodiments described hereinbefore. It will be understood that the benefits and advantages described above may relate to one embodiment or may relate to several embodiments. The embodiments are not limited to those that solve any or all of the stated problems or those that have any or all of the stated benefits and advantages. It will further be understood that reference to 'an' item refers to one or more of those items. The term "comprising" is used in this specification to mean including the feature(s) or act(s) followed thereafter, without excluding the presence of one or more additional features or acts.

## Claims

1. A method for determining whether a subject is at risk of developing or having atherosclerosis and/or a cardiovascular disease, wherein the method comprises
determining a quantitative value of the ability of cells obtained from a biological sample of the subject to take up low-density lipoprotein (LDL), a quantitative value of the expression of LDL receptor (LDLR) in the cells, a quantitative value of the lipid storage capability of the cells, and/or a quantitative value of the lipid mobilization capability of the cells;
wherein the quantitative value of the ability of the cells to take up LDL, the quantitative value of the expression of LDLR in the cells, the quantitative value of the lipid storage capability of the cells, and/or the quantitative value of the lipid mobilization capability of the cells is/are indicative of the likelihood of the subject having an increased risk of developing or having atherosclerosis and/or the cardiovascular disease.

2. The method according to claim 1, wherein the cells comprise or are peripheral blood mononuclear cells (PBMCs).

3. The method according to claim 1 or 2, wherein the cells comprise or are monocytes.

4. The method according to any one of claims 1 - 3, wherein the subject has hyperlipidemia.

5. The method according to any one of claims 1 - 4, wherein the cardiovascular disease comprises or is at least one of coronary artery disease, such as angina or myocardial infarction, stroke, heart attack, cerebrovascular disease, or peripheral artery disease.

6. The method according to any one of claims 1 - 5, wherein the method is performed using high content imaging followed by automated image and data analysis.

7. The method according to any one of claims 1 - 6, wherein the cells are immobilized on a surface, optionally as a monolayer.

8. The method according to any one of claims 1 - 7, wherein the cells are grouped to two or more cell populations, and the quantitative value of the ability to take up LDL, the quantitative value of the expression of LDLR, the quantitative value of the lipid storage capability, and/or the quantitative value of the lipid mobilization capability of at least one of the cell populations is/are determined separately from the other cell populations.

9. The method according to any one of claims 1 - 8, wherein the quantitative value of the ability of the cells of the subject to take up LDL is determined using labelled LDL particles, wherein the LDL particles are optionally fluorescently or radioactively labelled.

10. The method according to any one of claims 1 - 9, wherein the quantitative value of the ability of the cells of the subject to take up LDL is determined using LDL particles preserved by snap freezing.

11. The method according to any one of claims 1 - 10, wherein the quantitative value of the ability of the cells to take up LDL, the quantitative value of the expression of LDLR, the quantitative value of the lipid storage capability, and/or the quantitative value of the lipid mobilization capability is/are normalized using a standard comprising a mixture of cells obtainable from biological samples of a plurality of subjects.

12. The method according to any one of claims 1 - 11, wherein the method further comprises determining whether the subject is at risk of developing or having the cardiovascular disease on the basis of at least one genetic marker associated with the cardiovascular disease or the risk thereof, and/or the method further comprises calculating a risk score based on the presence of at least one genetic marker associated with the cardiovascular disease or the risk thereof and on the quantitative value of the ability of the cells to take up LDL, the quantitative value of the expression of LDLR, the quantitative value of the lipid storage capability, and/or the quantitative value of the lipid mobilization capability.

13. The method according to any one of claims 1 - 12, wherein
the method is performed using high content imaging followed by automated image and data analysis;
the cells are immobilized on a surface, optionally as a monolayer;
the cells are grouped to two or more cell populations, and the quantitative value of the ability to take up LDL, the quantitative value of the expression of LDLR, the quantitative value of the lipid storage capability, and/or the quantitative value of the lipid mobilization capability of at least one of the cell populations is/are determined separately from the other cell populations; and
the quantitative value of the ability of the cells to take up LDL, the quantitative value of the expression of LDLR, the quantitative value of the lipid storage capability, and/or the quantitative value of the lipid mobilization capability is/are normalized using a standard comprising a mixture of cells obtainable from biological samples of a plurality of subjects.

14. The method according to claim 13, wherein the quantitative value of the ability of the cells of the subject to take up LDL is determined using LDL particles preserved by snap freezing.

15. The method according to claim 13 or 14, wherein the cells comprise or are peripheral blood mononuclear cells (PBMCs), the PBMC cells are grouped to monocytes, lymphocytes and optionally to other cell types, and the quantitative value(s) are determined separately for the monocytes, lymphocytes, and any other optional cell populations.
